# FASCICULE DE BREVET EUROPEEN

(11) **EP 2 134 387 B1**
(45) Date de publication et mention de la délivrance du brevet: **31.10.2012**
(21) Numéro de dépôt: 08706392.1
(22) Date de dépôt: 07.03.2008
(51) Int. Cl.: A61M 5/14

(54) **UNITE DE POMPAGE DE NUTRITION ENTERALE OU PARENTERALE OU DE PERFUSION**
PUMPEINHEIT ZUR ENTERALEN ODER PARENTERALEN ERNÄHRUNG ODER PERFUSION
PUMPING UNIT FOR ENTERAL OR PARENTERAL NUTRITION OR PERFUSION

(30) Priorité: 12.03.2007 EP 07405082
(43) Date de publication de la demande: 23.12.2009
(73) Titulaire: Rochat, Jean-Denis, 1272 Genolier (CH)
(72) Inventeur: Rochat, Jean-Denis, 1272 Genolier (CH)
(86) Numéro de dépôt international: PCT/CH2008/000094
(87) Numéro de publication internationale: WO 2008/110024

(56) Documents cités:
- EP-A- 1 400 254
- EP-B1- 0 223 580
- WO-A-03/075984
- WO-A-2005/030299
- GB-A- 2 053 378
- US-A- 4 557 725

## Description

La présente invention se rapporte à une unité de pompage de nutrition entérale ou parentérale ou de perfusion comprenant une enceinte de pompage à usage unique munie d'un conduit d'admission, d'un conduit de refoulement et d'une membrane de pompage déformable de manière élastique, un poussoir pour déformer alternativement la membrane déformable ledit poussoir d'entraînement étant logé dans un boîtier-support dont une paroi présente une glissière de retenue pour venir en prise avec des éléments de coulissement de l'enceinte de pompage, ce boîtier-support présentant encore une ouverture de passage du poussoir d'entraînement, des moyens de butée pour déterminer la position de l'enceinte de pompage le long de la glissière de retenue et pour mettre ladite membrane de pompage en relation d'entraînement avec ledit poussoir d'entraînement.

La plupart des unités de pompage utilisées dans ce genre d'application font usage de pompes péristaltiques. L'inconvénient de ce genre de pompe provient de son très faible rendement dû aux frottements inhérents à sa conception. Par conséquent, il n'est pas possible d'avoir une autonomie de pompage supérieure à environ 3 litres avec une batterie d'alimentation de l'ordre de 26'000J. Cette faible autonomie constitue un inconvénient important, qui rend son utilisation ambulatoire problématique.

Un autre inconvénient de ces pompes vient de la mise en place de la tubulure entre les galets ou patins d'écrasement du tuyau et la surface d'appui cylindrique de ce tuyau par rapport à laquelle les galets ou patins sont entraînés. La pompe ne peut fonctionner que si le tuyau souple est posé correctement. Pour éviter tout risque de mise en place incorrecte, on propose en général de disposer la partie du tuyau souple qui coopère avec les galets de la pompe, ainsi que la surface d'appui contre laquelle le tuyau est écrasé dans une cassette qui comporte des moyens de fixation avec le dispositif d'entraînement des galets.

On a également proposé dans le US 5,647,852 une pompe à piston sous forme de cassette comprenant des moyens de positionnement et de fixation amovible sur un dispositif d'entraînement à moteur linéaire. Une telle pompe est utilisée pour le lavage dans le domaine médical, dentaire ou thérapeutique.

On a encore proposé dans le WO 2005/030299 un dispositif de pompage à cassette munie de membranes de commande et de pompage, dans lequel la cassette est introduite dans une glissière d'un chariot lui-même monté coulissant sur un châssis comportant le mécanisme d'entraînement de la cassette de pompage. Une fois la cassette mise en place sur le chariot, celui-ci est repoussé vers le châssis pour mettre les membranes de commande en contact avec des poussoirs d'entraînement. EP-A-1400 254 décrit une unité de pompage identique à celle du préambule de la revendication 1.

Le principal inconvénient de ce dispositif est sa complexité et donc son coût et sa fiabilité. En outre, la mise sous précontrainte de la membrane de pompage résulte du déplacement du chariot dans la même direction que celle de déplacement du poussoir de commande de cette membrane, mais en sens contraire. Donc, si la position du chariot en position repoussée vers le châssis n'est pas parfaitement exacte, la course de la membrane et donc le volume pompé seront variables.

Il n'existe actuellement pas, sur le marché, de pompe de nutrition entérale ou parentérale ou de perfusion susceptible de fonctionner à l'aide d'une batterie avec une grande autonomie. Les seules solutions connues utilisent des batteries spécifiques de plus de 20.000J, donc chères et qui n'ont une autonomie que de l'ordre de 3 litres, ce qui est trop peu pour rendre un tel système susceptible de satisfaire les exigences relatives à une utilisation ambulatoire ou à permettre de fournir une alternative valable aux systèmes classiques à entraînement du liquide par gravité et contrôle du débit par clamp. C'est une des raisons pour lesquelles l'entraînement du liquide par gravité est encore d'usage courant, malgré ses inconvénients.

Le but de la présente invention est de remédier, au moins en partie, aux problèmes posés par les systèmes de pompage susmentionnés.

A cet effet, cette invention a pour objet une unité de pompage de nutrition entérale ou parentérale ou de perfusion selon la revendication 1.

Compte tenu du type de pompe et de son dispositif d'entraînement, de préférence à course très réduite (typiquement < 2mm) et de préférence à fréquence élevée (typiquement autour de 10Hz) adoptés pour donner un débit de l'ordre de 2 1/h et une grande autonomie à l'unité de pompage objet de l'invention, il est important que le positionnement de la partie à usage unique sur le dispositif d'entraînement rende le nettoyage et la mise en place aussi facile que possible, avec une précision appropriée à un fonctionnement précis de l'unité de pompage.

Cette précision peut être obtenue par des éléments structurels, les dimensions respectives de la glissière recevant l'enceinte de pompage et celles de cette enceinte de pompage, qui sont parfaitement reproductibles du fait qu'une telle enceinte de pompage est produite par moulage par injection et que la précision est donnée par le moule. Quant à la précision de la glissière il s'agit d'un simple fraisage qui peut être réalisé lui aussi avec une grande précision. Le fait que la glissière se situe dans un plan perpendiculaire à la course du poussoir d'entraînement de la membrane de pompage contribue aussi à assurer une grande précision avec des moyens très simples.

Le dessin annexé illustre, schématiquement et à titre d'exemple, une forme d'exécution de l'unité de pompage objet de la présente invention.

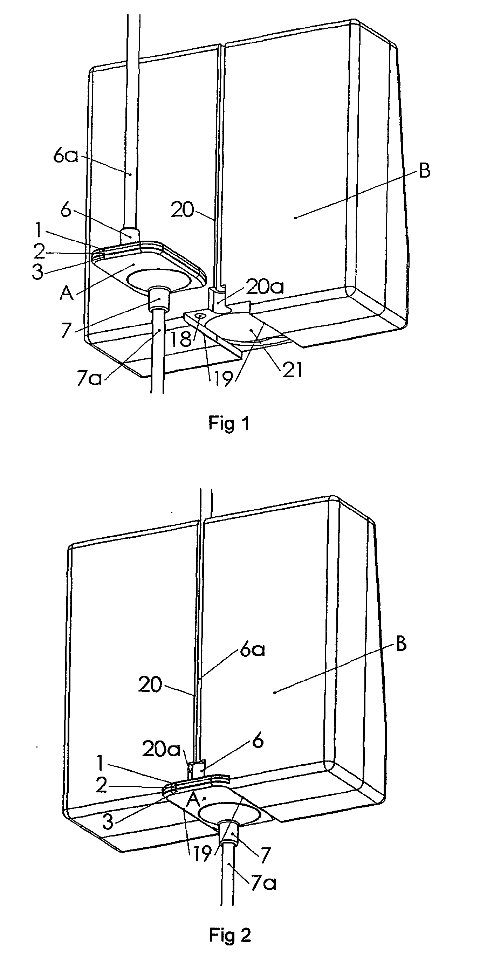

La figure 1 est une vue en perspective des deux parties séparées de l'unité de pompage;
la figure 2 est une vue en perspective des deux parties assemblées de l'unité de pompage;
la figure 3 est une vue en coupe selon la ligne de coupe III-III de la figure 2;
la figure 4 est une vue en coupe de la seule enceinte de pompage illustrée par la figure 3, avec la membrane de pompage libre.

L'unité de pompage objet de la présente invention comporte essentiellement deux parties, une pompe à usage unique A et un mécanisme d'entraînement de cette pompe situé dans un boîtier-support B.

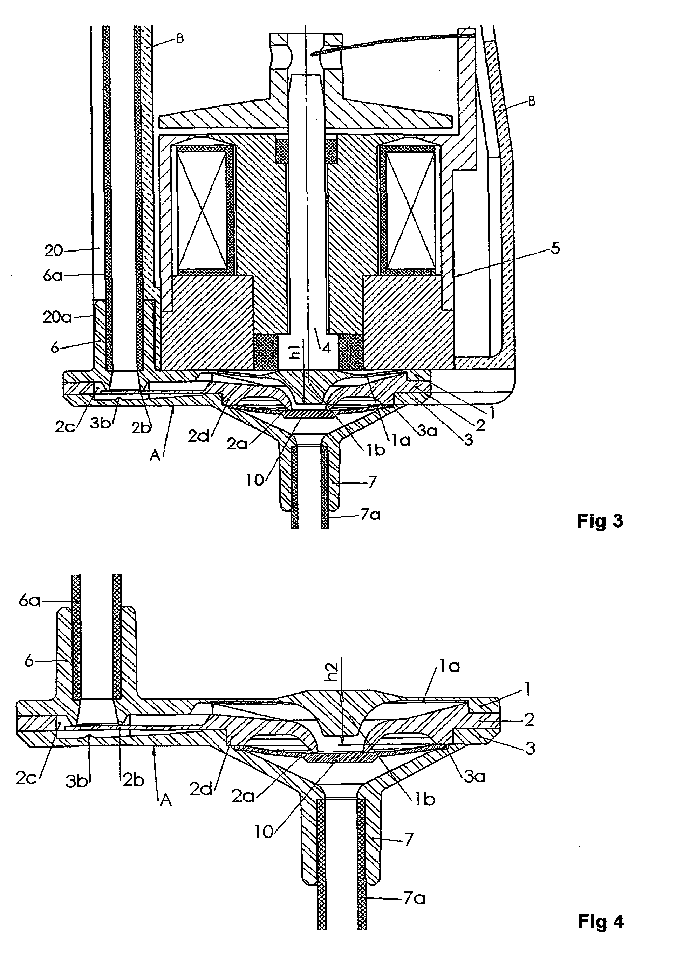

Dans l'exemple illustré, l'enceinte de pompage A à usage unique est une pompe dont l'élément de pompage est une membrane annulaire 1a dont une forme d'exécution est illustrée plus en détail par la figure 3. Comme on peut le constater, cette pompe à usage unique est essentiellement formée d'une enceinte en trois parties 1, 2, 3, deux parties 1, 3 formant la paroi de l'enceinte de pompage et une partie intermédiaire 2. Dans cet exemple, chacune des parties de paroi 1, 3 comporte un conduit d'admission 6 et un conduit de refoulement 7. La partie de paroi 1 présente une partie amincie formant une membrane annulaire 1a, entourant une partie d'actionnement plus épaisse 1b. La partie annulaire amincie 1a sert de membrane de pompage dont la partie centrale d'actionnement plus épaisse 1b sert à transmettre à la membrane annulaire la force exercée par un organe d'entraînement qui, dans cet exemple, est un poussoir formé par le noyau mobile 4 d'un électroaimant 5 d'entraînement de la pompe. La déformation de la membrane 1a doit évidemment rester dans les limites de déformation élastique de la matière plastique formant la partie de paroi 1.

La partie intermédiaire 2 comporte une ouverture de communication 2a pour mettre en communication sélective les compartiments amont et aval de la pompe. Cette ouverture de communication 2a est disposée en regard de la partie centrale épaisse 1b de la membrane et un clapet de valve 2b est situé en regard d'une ouverture 2c vis-à-vis de l'extrémité interne du conduit d'admission 6 ménagé dans la partie de paroi 1. L'ouverture 2a se situe à l'extrémité d'une dépression, tandis que la partie centrale d'actionnement plus épaisse 1b forme une saillie qui s'engage dans l'ouverture 2a. La partie intermédiaire 2 comporte encore une saillie annulaire 2d tournée vers la partie de paroi 3, dont le rôle sera expliqué ci-après.

La partie de paroi 3 comporte un siège concentrique au conduit de refoulement 7, pour le positionnement d'un clapet 10 de contrôle de l'ouverture de communication 2a de la partie intermédiaire, qui fait également office de dispositif contre l'écoulement libre, dans le but d'empêcher tout écoulement de liquide lorsque l'usage unique n'est pas inséré dans la pompe. Ce clapet 10 est disposé entre cette ouverture de communication 2a et le conduit de refoulement 7 de la pompe. Pour éviter que du liquide puisse s'écouler par gravité à travers la pompe, le clapet 10 est appliqué contre l'ouverture 2a avec une pression de 4.10⁴Pa ± 1.10⁴Pa.

En position de repos, il ferme l'ouverture 2a et est appliqué contre celle-ci dès que la différence de pression entre les côtés amont et aval de l'ouverture de communication 2a est inférieure à 4.10⁴Pa ± 1.10⁴Pa. Il s'écarte de cette ouverture 2a dès que la différence de pression sus-mentionnée est supérieure à 4.10⁴Pa ± 1.10⁴Pa.

La partie de paroi 3 présente un siège annulaire 3a pour le positionnement du clapet 10. Ce clapet 10 est retenu sur ce siège 3a par la saillie annulaire 2d de la partie intermédiaire 2. La partie de paroi 3 comporte encore une saillie 3b située en face du clapet 2b de contrôle du conduit d'admission 6, pour empêcher que ce clapet 2b ne vienne s'appliquer contre la face interne de la partie de paroi 3. Par cette disposition, la face du clapet 2b opposée à sa face adjacente à l'extrémité interne du conduit d'admission 6 de la pompe est exposée à la pression régnant dans le compartiment de la pompe situé en amont de l'ouverture de communication 2a de la partie intermédiaire 2. Ce clapet 2b peut ainsi fermer l'extrémité interne du conduit d'admission 6 en phase de refoulement de la pompe et l'ouvrir en phase d'aspiration.

Avantageusement, la pompe décrite ci-dessus est conçue pour fonctionner avec une grande autonomie. A cet effet, on a réuni un certain nombre de conditions, aussi bien en ce qui concerne la pompe proprement dite que son dispositif d'entraînement. Les détails relatifs à cette pompe et à son dispositif d'entraînement sont décrits dans la demande de brevet EP 07405078.2 à laquelle on pourra se reporter pour plus de détails, ceux-ci n'étant pas nécessaire pour comprendre la présente invention.

Tout d'abord, au niveau de la pompe proprement dite, la membrane 1a a un diamètre relativement petit compris entre 3 et 25mm, avantageusement situé autour de 16mm de manière à limiter sa force d'actionnement qui est le produit de la pression P par la surface S. Etant donné que l'on envisage de préférence d'actionner cette membrane par un électro-aimant de faible consommation, on a choisi un électroaimant de faible dimension. La course de la membrane 1a, entraînée par le noyau-poussoir 4 de l'électro-aimant, se situe de préférence entre 0,2 et 2mm, avantageusement de l'ordre de 0,5mm. Dans ces conditions, la course de la membrane 1a permet son entraînement direct par le noyau-poussoir 4 de l'électro-aimant et évite tout réducteur mécanique qui diminue sensiblement le rendement global de la pompe.

Avantageusement, l'épaisseur de la membrane élastique 1a se situe entre 0,1 et 0,7mm, de préférence de l'ordre de 0,3mm. Ces dimensions permettent d'utiliser le même matériau thermoplastique pour la membrane 1a que pour la partie 1 de paroi de l'enceinte de la pompe, ce qui permet de réaliser la partie 1 et la membrane 1a en une seule opération d'injection. Parmi les matériaux thermoplastiques utilisables, on peut citer le PC, le PVC, L'ABS, le PP ou le PE notamment. Le choix est fonction du coût, de la précision et de la stabilité des caractéristiques élastiques après stérilisation et stockage sur une durée maximum de trois ans. Le PC est le matériau qui correspond le mieux à ce cahier des charges.

Pour économiser au maximum l'énergie nécessaire à l'entraînement de la pompe, on a prévu d'utiliser l'élasticité de la membrane 1a de la pompe pour la ramener à sa position de repos après son entraînement par le noyau mobile 4 de l'électroaimant 5, en sorte que cette membrane 1a doit être mise sous une précontrainte, avantageusement de l'ordre de 2.10⁴Pa.

A cet effet, la face inférieure du boîtier-support B de l'électroaimant d'entraînement 5 comporte une glissière de retenue 19, qui s'étend sur toute la largeur de la face inférieure du boîtier-support B et dont le profil de la section droite peut être avantageusement en queue d'aronde formant ainsi deux patins de coulissement à environ 45° pour venir en prise avec deux bords parallèles des parois 1, 3 de l'enceinte de pompage A, qui constituent les éléments de coulissement de cette enceinte de pompage A. Cette glissière de retenue 19 peut présenter tout autre profil approprié, adapté à celui des bords parallèles des parois 1, 3 de l'enceinte de pompage A, pour permettre de plaquer et maintenir la face externe de la paroi 1 de l'enceinte de pompage A à usage unique appliquée contre le fond de la glissière de retenue 19. Comme on le voit sur la figure 3, lorsque l'enceinte de pompage A est en prise avec la glissière 19, la membrane déformable 1a est soumise à une déformation de précontrainte en position de repos, afin de lui permettre de revenir suffisamment rapidement à sa position de repos par sa propre élasticité après avoir été déplacée par le noyau-poussoir 4 de l'électroaimant 5.

Si on compare la membrane déformable 1a précontrainte de la figure 3 avec celle de la figure 4 où la membrane déformable 1a est à l'état libre, on constate que la différence entre la hauteur h₂ de la membrane 1a de la figure 4 et la hauteur h₁ de la même membrane précontrainte de la figure 3 donne la valeur de la précontrainte. Cette précontrainte résulte exclusivement de la différence entre la profondeur de la glissière 19 qui correspond à h₁ et la hauteur h₂ qui correspond à la partie de la hauteur de l'enceinte de pompage A placée dans la glissière 19. Cette précontrainte, ainsi que la précision de la course de la membrane déformable 1a induite par le poussoir 4 sont donc exclusivement données par les dimensions constructives et non par la précision de la course de l'enceinte de pompage A dans la glissière 19.

La seule introduction de l'enceinte de pompage A dans la glissière 19 garantit une précision parfaitement reproductible. En plus, la glissière constitue un élément aisément accessible et visible permettant de garantir sa propreté et la pérennité de la précision au cours du temps.

Une ouverture de communication 21 est ménagée dans le fond de la glissière de retenue 19 pour permettre au noyau-poussoir 4 de l'électroaimant 5 de venir en prise avec la partie centrale plus épaisse 1b de l'enceinte de pompage A. On peut encore constater que la glissière de retenue 19 traverse toute la largeur de la paroi inférieure du boîtier-support B, ce qui facilite son nettoyage du fait que cette glissière de retenue débouche vers l'extérieur à ses deux extrémités, il n'existe pas de recoin dans lequel de 1a poussière pourrait s'accumuler.

Le positionnement et le maintien de l'enceinte de pompage A à usage unique dans la glissière de retenue 19 sont obtenus par clipage du conduit d'aspiration souple 6a ou de celui de refoulement 7a de l'enceinte de pompage A à usage unique dans une goulotte 20 ménagée dans une paroi du boîtier-support B adjacente à la paroi inférieure dans laquelle est ménagée la glissière de retenue et dont la largeur de l'ouverture longitudinale donnant accès à cette goulotte 20 est légèrement plus étroite que le diamètre du conduit 6a ou 7a, en sorte qu'une légère déformation élastique de la section de ce conduit 6a ou 7a est nécessaire pour l'introduire dans la goulotte 20 après quoi, la section du conduit souple 6a ou 7a reprend sa forme initiale, garantissant le maintien de l'enceinte de pompage A dans la glissière de retenue 19 et le positionnement de la partie centrale plus épaisse 1b de la membrane 1a vis-à-vis du noyau-poussoir 4 de l'électroaimant 5. La goulotte 20 présente à sa base une partie 20a de plus grand diamètre pour recevoir la partie 6 du conduit d'admission solidaire de 1a pompe A.

Pour éviter l'usure des patins de coulissement de la glissière de retenue 19, on peut avantageusement renforcer le plastique du boîtier-support B par des billes de verre de 50µm à 500µm. Ce renforcement permet aussi de protéger le boîtier-support des rayures.

Un interrupteur ON-OFF 18 est disposé dans le fond de la glissière 19 pour permettre d'enclencher l'électro-aimant 5 seulement lorsque la pompe A est engagée dans la glissière 19. L'électroaimant 5 se met alors en mode prêt à pomper. L'utilisateur peut ensuite introduire les paramètres de commande de la pompe à l'aide d'un poste de commande (non représenté). Cet interrupteur 18 pourrait aussi se situer dans la goulotte 20 ou dans sa partie élargie 20a, ce qui aurait le même effet de mettre l'électroaimant en mode prêt à pomper que lorsque la pompe A est en place.

## Revendications

1. Unité de pompage de nutrition entérale ou parentérale ou de perfusion comprenant une enceinte de pompage à usage unique (A) munie d'un conduit d'admission (6), d'un conduit de refoulement (7) et d'une membrane de pompage (1a) déformable de manière élastique, un poussoir (4) pour déformer alternativement la membrane déformable (1a) ledit poussoir d'entraînement étant logé dans un boîtier-support (B) dont une paroi présente une glissière de retenue (19) pour venir en prise avec des éléments de coulissement de l'enceinte de pompage (A), ce boîtier-support (B) présentant encore une ouverture de passage (21) du poussoir d'entraînement (4), des moyens de butée (20) pour déterminer la position de l'enceinte de pompage (A) le long de la glissière de retenue (19) et pour mettre ladite membrane de pompage (1a) en relation d'entraînement avec ledit poussoir d'entraînement (4), **caractérisée en ce que** la profondeur de la glissière de retenue (19) recevant l'enceinte de pompage (A) est inférieure à la hauteur entre les éléments de coulissement de l'enceinte de pompage (A) et la face externe de la membrane de pompage (1a), de manière que la mise en place de l'enceinte de pompage (A) dans la glissière de retenue (19) provoque automatiquement une mise sous précontrainte déterminée de la membrane de pompage (1a).

2. Unité de pompage selon la revendication 1, comprenant des moyens de fixation amovible (19, 20) de ladite enceinte de pompage (A) dans ladite position déterminée le long de la glissière de retenue (19).

3. Unité de pompage selon l'une des revendications précédentes, dans laquelle une paroi du boîtier-support (B) adjacente à la paroi qui présente 1a glissière de retenue (19) comporte une goulotte (20) de clipage de l'un des conduits d'admission (6) ou de refoulement (7), cette goulotte (20) de clipage servant à définir la position de ladite membrane déformable (1a) par rapport au poussoir d'entraînement (4) et des moyens de fixation amovible de l'enceinte de pompage le long de la glissière de retenue (19).

4. Unité de pompage selon l'une des revendications précédentes, dans laquelle un interrupteur ON-OFF (18), de mise du mécanisme d'entraînement (5) en mode prêt à pomper, est disposé sur le boîtier-support (B) à un endroit où il peut être actionné par la mise en place de l'enceinte de pompage (A) dans la glissière de retenue (19) .

## Claims

1. Pumping unit for enteral, parenteral or infusion feeding, comprising a single-use pumping chamber (A) provided with an inlet duct (6), a delivery duct (7) and a resiliently deformable pumping membrane (1a), a pusher (4) for alternately deforming the deformable membrane (1 a), said driving pusher being housed in a support casing (B), one wall of which has a retaining slideway (19) with which the sliding elements of the pumping chamber (A) engage, this support casing (B) also having a through-opening (21) for the driving pusher (4), and abutment means (20) for determining the position of the pumping chamber (A) along the retaining slideway (19) and for putting said pumping membrane (1a) into drive connection with said driving pusher (4), **characterised in that** the depth of the retaining slideway (19) holding the pumping chamber (A) is smaller than the height between the sliding elements of the pumping chamber (A) and the external surface of the pumping membrane (1a), such that installing the pumping chamber (A) in the retaining slideway (19) automatically puts the pumping membrane (1a) under a predetermined preload.

2. Pumping unit according to claim 1, comprising means (19, 20) for removably fastening said pumping chamber (A) in said predetermined position along the retaining slideway (19).

3. Pumping unit according to any of the preceding claims, wherein a wall of the support casing (B) adjacent to the wall with the retaining slideway (19) has a channel (20) for clipping in either the inlet duct (6) or the delivery duct (7), this clip-in channel (20) defining the position of said deformable membrane (1a) with respect to the driving pusher (4) and the means for removably fastening the pumping chamber along the retaining slideway (19).

4. Pumping unit according to any of the preceding claims, wherein an ON-OFF switch (18) for putting the drive mechanism (5) into the ready-to-pump mode is located on the support casing (B) at a location where it can be actuated by the installation of the pumping chamber (A) in the retaining slideway (19).

## Patentansprüche

1. Pumpeinheit zur enteralen oder parenteralen Ernährung oder zur Infusion mit einem Einwegpumpgehäuse (A), das einen Einlasskanal (6), einen Auslasskanal (7) und eine elastisch verformbare Pumpmembran (1a) aufweist, mit einem Stößel (4) zum abwechselnden Verformen der verformbaren Membran (1a), wobei der Antriebsstößel in einem Trägergehäuse (B) angeordnet ist, bei dem eine Wand eine Rückhalteschiene (19) aufweist, um mit den Gleitelementen des Pumpgehäuses (A) in Eingriff zu kommen, wobei das Trägergehäuse (B) weiterhin eine Durchgangsöffnung (21) für den Antriebsstößel (4) aufweist, mit Anschlagsmitteln (20) zum Festlegen der Stellung des Pumpgehäuses (A) entlang der Rückhalteschiene (19) und um die Pumpmembran (1a) in ein Antriebsverhältnis mit dem Antriebsstößel (4) zu bringen, **dadurch gekennzeichnet, dass** die Tiefe der Rückhalteschiene (19), die das Pumpgehäuse (A) aufnimmt, kleiner als die Höhe zwischen den Gleitelementen des Pumpgehäuses (A) und der Außenseite der Pumpmembran (1a) ist, so dass das Anordnen des Pumpgehäuses (A) in die Rückhalteschiene (19) die Pumpmembran (1a) automatisch unter eine bestimmte Vorspannung setzt.

2. Pumpeinheit nach Anspruch 1, die Mittel für eine lösbare Befestigung (19, 20) des Pumpgehäuses (A) in der festgelegten Stellung entlang der Rückhalteschiene (19) aufweist.

3. Pumpeinheit nach einem der vorangehenden Ansprüche, bei der eine Wand des Trägergehäuses (B), die benachbart zu der die Rückhalteschiene (19) aufweisenden Wand angeordnet ist, eine Rinne (20) für das Einrasten entweder des Einlasskanals (6) oder des Auslasskanals (7) aufweist, wobei diese Rinne (20) für das Einrasten dazu dient, die Stellung der verformbaren Membran (1a) in Bezug auf den Antriebsstößel (4) und der Mittel für die lösbare Befestigung des Pumpgehäuses entlang der Rückhalteschiene (19) festzulegen.

4. Pumpeinheit nach einem der vorangehenden Ansprüche, bei der ein EIN-AUS-Schalter (18), um den Antriebsmechanismus (5) in einen Modus zu bringen, in dem er zum Pumpen bereit ist, auf dem Trägergehäuse (B) an einer Stelle angebracht ist, wo er durch das Anordnen des Pumpgehäuses (A) in der Rückhalteschiene (19) betätigbar ist.
